# FASCICULE DE BREVET EUROPEEN

(11) **EP 2 391 381 B1**
(45) Date de publication et mention de la délivrance du brevet: **07.10.2015**
(21) Numéro de dépôt: 10707062.5
(22) Date de dépôt: 28.01.2010
(51) Int. Cl.: A61K 38/40, A61K 33/18, A61P 11/00

(54) **UTILISATION D'UNE ASSOCIATION SYNERGIQUE D'IONS HYPOTHIOCYANITES ET/OU HYPOHALITES ET DE LACTOFERRINE POUR LA PRÉPARATION POUR LE TRAITEMENT DE LA MUCOVISCIDOSE**
VERWENDUNG EINER SYNERGISTISCHEN KOMBINATION VON HYPOTHIOCYANIT UND/ODER HYPOHALIT-IONEN UND LACTOFERRIN ZUR HERSTELLUNG EINER BEHANDLUNG GEGEN ZYSTISCHE FIBROSE
USE OF A SYNERGISTIC COMBINATION OF HYPOTHIOCYANITE AND/OR HYPOHALITE IONS AND LACTOFERRIN FOR PREPARING A TREATMENT FOR CYSTIC FIBROSIS

(30) Priorité: 28.01.2009 FR 0950536
(43) Date de publication de la demande: 07.12.2011
(73) Titulaire: Alaxia, 69008 Lyon (FR)
(72) Inventeur: BORDEAU, Philippe, F-69330 Meyzieu (FR); PERRAUDIN, Jean-Paul, B-1180 Bruxelles (BE)
(74) Mandataire: KATZAROV S.A.
(86) Numéro de dépôt international: PCT/FR2010/000070
(87) Numéro de publication internationale: WO 2010/086530

(56) Documents cités:
- WO-A-2005/113026
- WO-A-2007/134180
- WO-A-2008/003688
- SANDRE-BALLESTER ET AL: "A gift of Nature for the treatment of Pseudomonas aeruginosa" JOURNAL OF CYSTIC FIBROSIS, ELSEVIER LNKD- DOI:10.1016/S1569-1993(09)60116-8, vol. 8, 1 juin 2009 (2009-06-01), page S29, XP026192287 ISSN: 1569-1993 [extrait le 2009-06-01]
- TRAVIS SUE M ET AL: "Activity of abundant antimicrobials of the human airway" AMERICAN JOURNAL OF RESPIRATORY CELL AND MOLECULAR BIOLOGY, vol. 20, no. 5, mai 1999 (1999-05), pages 872-879, XP002534861 ISSN: 1044-1549

## Description

La mucoviscidose est une maladie génétique grave, la mutation affecte la protéine CFTR et entraîne une anomalie de transfert, notamment des ions chlorures.

La viscosité du mucus est augmentée, il obstrue les bronches et bloque les sécrétions enzymatiques digestives. L'expression clinique de la maladie comporte un syndrome respiratoire et un syndrome digestif.

Une des principales causes de mortalité est due à la colonisation progressive des poumons par des micro-organismes tels que les Pseudomonas aeruginosa et/ou le Burkholderia cepacia ou encore le staphylocoque doré qui agressent les poumons et détruisent les capacités respiratoires des malades.

Des travaux scientifiques récents des Docteurs Banfi, dans Am. J. Respir. Crit. Care Med., 2007, 175(9) : 967, Conner et Childers, ont mis en évidence l'absence de la production de l'ion hypothiocyanite OSCN- chez les personnes atteintes de mucoviscidose. Celui-ci est présent naturellement dans la salive et les sécrétions pulmonaires et il participe au système immunitaire. Son absence pourrait expliquer l'extrême sensibilité des poumons des personnes atteintes.

L'ion hypothiocyanite et/ou hypohalite est généré notamment in vivo par le système lactoperoxidase selon l'équation ci-dessous :

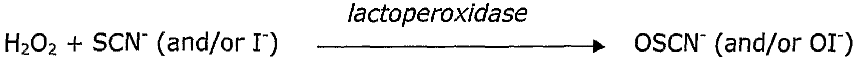

Les propriétés pharmacologiques de l'ion hypothiocyanite sont connues notamment ses propriétés biocides, mais en raison de l'instabilité de cette espèce chimique dont la demi-vie est d'environ 24 heures, aucune formulation permettant un traitement local pulmonaire dans des conditions satisfaisantes n'a pu être développée.

On connait par exemple de WO2007134180, une composition thérapeutique agissant par action de l'ion hypothiocyanite, comprenant un système enzymatique, par exemple une oxydoréductase qui par réduction d'un substrat spécifique produit du peroxyde d'hydrogène, le substrat spécifique, par exemple du glucose, l'ion SCN⁻, et la lactoperoxidase. On comprend la difficulté de formulation de telles compositions thérapeutiques et les effets secondaires susceptibles d'être générés par exemple par la production in vivo, ici dans les voies respiratoires de peroxyde d'hydrogène, qui a un effet inflammatoire et génotoxique et ne peut être administré dans des traitements chroniques.

Dans US2002/172645, l'ion thiocyanate est administré seul pour alimenter le système lactoperoxidase endogène et former des ions hypothiocyanites in vivo ou comme dans WO2007134180 en combinaison avec le système lactoperoxydase.

On connait de WO2008/003688 la démonstration d'une synergie entre l'ion hypothiocyanite et la lactoferrine.

Les propriétés de la lactoferrine sont par ailleurs bien connues et notamment son action sur les biofilms et son action anti-inflammatoire.

Cependant à l'heure actuelle aucune formulation satisfaisante permettant un traitement local et notamment la destruction des bactéries qui se développent sur le mucus des patients atteints de mucoviscidose n'a été développée et en particulier sur les *Burkholderia cepacia,* très pathogènes et particulièrement difficiles à éradiquer.

L'invention concerne l'utilisation d'une association synergique d'au moins un ion sélectionné dans le groupe des hypothiocyanites (OSCN⁻) et/ou des hypohalites et de lactoferrine pour la préparation d'une composition pharmaceutique pour le traitement de la mucoviscidose et des infections pulmonaires associées.

Dans un mode de réalisation, l'invention concerne l'utilisation d'une association synergique d'au moins un ion sélectionné dans le groupe des hypothiocyanites (OSCN⁻) et/ou des hypohalites et de lactoferrine pour la préparation d'une composition pharmaceutique pour le traitement des infections pour le traitement de la mucoviscidose et des infections pulmonaires associées provoquées par au mois une bactérie choisie dans le groupe constitué par *Burkholderia cepacia, Pseudomoas aeruginosa* et *Staphylococcus aureus.*

Dans un mode de réalisation, elle concerne les infections pulmonaires associées provoquées par *Burkholderia cepacia.*

Dans un mode de réalisation, l'ion est l'ion hypothiocyanite (OSCN⁻).

Les ions hypohalites sont choisis dans le groupe constitué par les ions hypoiodite, hypochlorite et ou hypobromite.

Dans un mode de réalisation, l'ion est l'ion hypoiodite (OI⁻).

Dans un autre mode de réalisation, la lactoferrine est une lactoferrine d'une pureté supérieure à 95%, substantiellement exempte d'endotoxine, de lipopolysaccharide et d'angiogénine et de avec un niveau de saturation en Fer supérieur à 15 %.

L'invention concerne également une utilisation dans une méthode de traitement thérapeutique de la mucoviscidose caractérisée en ce qu'elle comprend l'administration pour un traitement local de l'épithélium pulmonaire d'une quantité thérapeutiquement active d'une association synergique d'au moins un ion sélectionné dans le groupe des hypothiocyanites et/ou des hypohalites et de lactoferrine.

En effet dans la mucoviscidose les bactéries se développent sur l'épithélium des poumons et le traitement doit être local, l'administration sera donc effectuée par voie buccale et/ou nasale et/ou tout autre voie artificielle permettant d'atteindre le poumon, par exemple trachéotomie.

Dans un mode de réalisation, l'ion est l'ion hypothiocyanite (OSCN⁻).

Dans un mode de réalisation, l'ion est l'ion hypoiodite (OI⁻).

Dans un autre mode de réalisation, la lactoferrine est une lactoferrine d'une pureté supérieure à 95%, substantiellement exempte de lipopolysaccharide, d'endotoxine et d'angiogénine et avec un niveau de saturation en Fer supérieur à 15 %.

Les formulations selon l'invention agissent par les mécanismes suivants :
- la lactoferrine détruit le biofilm
- l'association lactoferrine/OSCN⁻ détruit les bactéries et/ou empêche leur croissance, elle a donc un effet bactériostatique et bactéricide.

L'association de la lactoferrine avec l'ion hypothiocyanite permet d'une part de réduire la concentration en hypothiocyanite pour atteindre la même efficacité antimicrobienne, d'autre part d'ajouter à l'aspect antimicrobien, l'aspect anti-inflammatoire. Enfin, la lactoferrine agit comme fluidifiant vis à vis des expectorats qui sont un des problèmes majeurs dans cette pathologie.

L'invention concerne également une formulation pharmaceutique destinée au traitement des phases aigües de la mucoviscidose caractérisée en ce qu'elle comprend 500 µM d'ion OSCN⁻ et 20 mg de Lactoferrine.

L'invention concerne également une formulation pharmaceutique destinée au traitement des phases aigües de la mucoviscidose caractérisée en ce qu'elle comprend 250 µM d'ion OSCN⁻ et 10 mg de Lactoferrine.

L'invention concerne également une formulation pharmaceutique destinée au traitement chronique de la mucoviscidose caractérisée en ce qu'elle comprend 25 µM d'ion OSCN⁻ et 1 mg de Lactoferrine.

L'invention concerne également un mode d'administration d'une formulation selon l'invention selon un schéma posologique caractérisé en ce qu'il comprend l'administration deux fois par jour de 5 ml d'une formulation comprenant 500 µM d'ion OSCN- et 20 mg de Lactoferrine dans les phases aigües de la mucoviscidose

L'invention décrit également un mode d'administration d'une formulation selon l'invention selon un schéma posologique caractérisé en ce qu'il comprend l'administration deux fois par jour de 5 ml d'une formulation comprenant 250 µM d'ion OSCN- et 10 mg de Lactoferrine dans les phases aigües de la mucoviscidose.

L'invention concerne également un mode d'administration d'une formulation selon l'invention selon un schéma posologique caractérisé en ce qu'il comprend l'administration deux fois par jour de 5 ml d'une formulation comprenant 25 µM d'ion OSCN- et 1 mg de Lactoferrine en traitement de fond chronique.

### Exemples :

Une composition extemporanée de traitement journalier est réalisée à l'aide d'un dispositif portable autonome, elle comprend 250 µM d'ion OSCN⁻, 2,4 mM d'ion SCN⁻ et 2,6 mM de lactoferrine.

La préparation ne contient ni glucose oxydase, ni lactoperoxydase, ni peroxyde d'hydrogène (la teneur est mesurée inférieure à 1 ppm).

Cette préparation pourra être administrée par voie aérienne à l'aide d'un pulvérisateur et/ou nébulisateur et/ou aérosoliseur, à raison de 1 ml à 5 ml de solution par inhalation (exemple : inhalation d'une solution de 2 ml en 20 minutes) de façon à atteindre les cibles au niveau pulmonaires.

### Résultats pharmacologiques

La solution de l'exemple a été testée in vitro sur une souche mucoïde de *Pseudomonas aeruginosa, Burkholderia cepacia, Methycillin Resistant Staphylococcus Aureus.*

Burkholderia cenocepacia J2315 (ATCC BAA 245 connue pour être résistante à Tobramycine et Colistin (Holden 2009, Soiza 2004)), *issue de patients ayant la mucoviscidose.*

### Pseudomonas aeruginosa

### Temps de contact en heures

| | 0 | 0,5 | 1 | 2 | 4 | 6 | 24 |
|---|---|---|---|---|---|---|---|
| Contrôle | 4,14 | 4,15 | 4,16 | 4,17 | 4,16 | 4,16 | 4,13 |
| OSCN 500µM | 4,14 | 3,23 | 3,16 | 2,98 | 2,08 | 1,90 | 1,78 |
| OSCN 250 µM + Lactoferrine | 4,14 | 2,93 | 2,89 | 2,82 | 2,15 | 1,79 | 1,51 |

| | | | | | | | |
|---|---|---|---|---|---|---|---|
| *(Résultats exprimés en log CFU*/*ml)* | | | | | | | |

### Burkholderia cepacia

| | Temps de contact en heures | | | | | | |
|---|---|---|---|---|---|---|---|
| | 0 | 0,5 | 1 | 2 | 4 | 6 | 24 |
| Contrôle | 4,41 | 4,47 | 4,48 | 4,54 | 4,56 | 4,58 | 4,53 |
| OSCN 500µM | 4,41 | 3,64 | 3,56 | 3,03 | 2,08 | 1,98 | 1,78 |
| OSCN 250 µM + | 4,41 | 3,42 | 3,50 | 2,99 | 2,00 | 1,84 | 1,56 |

| | | | | | | | |
|---|---|---|---|---|---|---|---|
| *Staphylococcus aureus résistant à la méthyciline (MRSA)* | | | | | | | |

| | Temps de contact en heures | | | | | | |
|---|---|---|---|---|---|---|---|
| | 0 | 0,5 | 1 | 2 | 4 | 6 | 24 |
| Contrôle | 4,15 | 4,17 | 4,18 | 4,17 | 4,22 | 4,20 | 4,24 |
| OSCN 500µM | 4,15 | 3,80 | 3,70 | 3,33 | 2,89 | 2,25 | 1,97 |
| OSCN 250 µM + Lactoferrine | 4,15 | 3,63 | 3,60 | 3,21 | 2,72 | 2,08 | 1,66 |

| | | | | | | | |
|---|---|---|---|---|---|---|---|
| *(Résultats exprimés en log CFU*/*ml)* | | | | | | | |

La solution de l'exemple a été testée in vivo sur souris contaminée avec une souche mucoïde de Pseudomonas aeruginosa.

| | Contrôle | Traité |
|---|---|---|
| Poumon de souris 72h après infection | 3,1 | 1,5 |

| | | |
|---|---|---|
| *Résultats exprimés en log CFU*/*ml* | | |

Les résultats permettent de démontrer une véritable action biocide et bactéricide vis-à-vis des souches mucoïdes testées et la diminution des doses d'ions hypothiocyanite à utiliser.

## Revendications

1. Composition comprenant une association synergique d'au moins un ion sélectionné dans le groupe des hypothiocyanites et/ou des hypohalites et de lactoferrine pour son utilisation dans le traitement de la mucoviscidose et des infections pulmonaires associées.

2. Composition pour son utilisation dans le traitement de la mucoviscidose et des infections pulmonaires associées selon la revendication 1, **caractérisée en ce que** les infections pulmonaires associées sont provoquées par au moins une bactérie choisie dans le groupe constitué par *Burkholderia cepacia, Pseudomonas aeruginosa* et *Staphylococcus aureus.*

3. Composition pour son utilisation dans le traitement de la mucoviscidose et des infections pulmonaires associées selon l'une quelconque des revendications précédentes, **caractérisée en ce que** l'ion est l'ion hypothiocyanite (OSCN⁻).

4. Composition pour son utilisation dans le traitement de la mucoviscidose et des infections pulmonaires associées selon l'une quelconque des revendications précédentes, **caractérisée en ce que** lion est l'ion hypoiodite (OI⁻)

5. Composition pour son utilisation dans le traitement de la mucoviscidose et des infections pulmonaires associées selon l'une des revendications précédentes pour son utilisation dans le traitement de la mucoviscidose **caractérisée en ce que** ledit traitement comprend l'administration pour un traitement local de l'épithélium pulmonaire d'une quantité thérapeutiquement active d'une association synergique d'au moins un ion sélectionné dans le groupe des hypothiocyanites et/ou des hypohalites et de lactoferrine.

6. Formulation pharmaceutique destinée au traitement des phases aigües de la mucoviscidose **caractérisée en ce qu'**elle comprend 500 µM d'ion OSCN⁻ et 20 mg de Lactoferrine.

7. Formulation pharmaceutique destinée au traitement des phases aigües de la mucoviscidose **caractérisée en ce qu'**elle comprend 250 µM d'ion OSCN⁻ et 10 mg de Lactoferrine.

8. Formulation pharmaceutique destinée au traitement chronique de la mucoviscidose **caractérisée en ce qu'**elle comprend 25 µM d'ion OSCN⁻ et 1 mg de Lactoferrine.

9. Composition pour son utilisation dans le traitement de la mucoviscidose et des infections pulmonaires associées selon l'une des revendications 1 à 6, **caractérisée en ce que** ledit traitement est effectué selon un schéma posologique qui comprend l'administration deux fois par jour de 5 ml d'une formulation comprenant 250 µM d'ion OSCN⁻ et 10 mg de Lactoferrine pendant un durée de quatre semaines dans les phases aigües de la mucoviscidose.

10. Composition pour son utilisation dans le traitement de la mucoviscidose et des infections pulmonaires associées selon l'une des revendications 1 à 6, **caractérisée en ce que** ledit traitement est effectué selon un schéma posologique qui comprend l'administration deux fois par jour de 5 ml d'une formulation comprenant 500 µM d'ion OSCN⁻ et 20 mg de Lactoferrine pendant un durée de quatre semaines dans les phases aigües de la mucoviscidose

11. Composition pour son utilisation dans le traitement de la mucoviscidose et des infections pulmonaires associées selon l'une des revendications 1 à 6, **caractérisée en ce que** ledit traitement est effectué selon un schéma posologique qui comprend l'administration deux fois par jour de 5 ml d'une formulation comprenant 25 µM d'ion OSCN⁻ et 1 mg de Lactoferrine en traitement de fond chronique.

## Patentansprüche

1. Zusammensetzung mit einer synergistischen Kombination von mindestens einem Ion, ausgewählt aus der Gruppe von Hypothiocyaniten und/oder Hypohaliten und Lactoferrin zur Verwendung in der Behandlung von zystischer Fibrose und assoziierten Lungeninfektionen.

2. Zusammensetzung zur Verwendung in der Behandlung von zystischer Fibrose und assoziierten Lungeninfektionen gemäss Anspruch 1, **dadurch gekennzeichnet, dass** die assoziierten Lungeninfektionen ausgelöst werden durch mindestens eine Bakterie ausgewählt aus der Gruppe bestehend aus *Burkholderia cepacia, Pseudomonas aeruginosa* und *Staphylococcus aureus.*

3. Zusammensetzung zur Verwendung in der Behandlung von zystischer Fibrose und assoziierten Lungeninfektionen gemäss irgendeinem der vorhergehenden Ansprüche, **dadurch gekennzeichnet, dass** das Ion das Hypothiocyanition (OSCN⁻) ist.

4. Zusammensetzung zur Verwendung in der Behandlung von zystischer Fibrose und assoziierten Lungeninfektionen gemäss irgendeinem der vorhergehenden Ansprüche, **dadurch gekennzeichnet, dass** das Ion das Hypohalition (OI⁻) ist.

5. Zusammensetzung zur Verwendung in der Behandlung von zystischer Fibrose und assoziierten Lungeninfektionen gemäss irgendeinem der vorhergehenden Ansprüche, **dadurch gekennzeichnet, dass** die besagte Behandlung die Abgabe einer therapeutisch wirksamen Menge einer synergistischen Kombination von mindestens einem Ion ausgewählt aus der Gruppe der Hypothiocyaniten und/oder Hypohaliten und Lactoferrin für eine Lokalbehandlung von Lungenpithel umfasst.

6. Pharmazeutische Formulierung, bestimmt für die Behandlung von akuten Phasen der zystischen Fibrose, **dadurch gekennzeichnet, dass** sie 500µM an OSCN⁻-Ion und 20mg an Lactoferrin umfasst.

7. Pharmazeutische Formulierung, bestimmt für die Behandlung von akuten Phasen der zystischen Fibrose, **dadurch gekennzeichnet, dass** sie 250µM an OSCN⁻-Ion und 10mg an Lactoferrin umfasst.

8. Pharmazeutische Formulierung bestimmt für die Behandlung von akuten Phasen der zystischen Fibrose, **dadurch gekennzeichnet, dass** sie 25µM an OSCN⁻-Ion und 1mg an Lactoferrin umfasst.

9. Zusammensetzung zur Verwendung in der Behandlung von zystischer Fibrose und assoziierten Lungeninfektionen gemäss einem der Ansprüche 1 bis 6, **dadurch gekennzeichnet, dass** die besagte Behandlung gemäss einem Dosierungsschema durchgeführt wird, welches die zweimal tägliche Abgabe von 5 ml einer Formulierung mit 250µM an OSCN⁻-Ion und 10mg an Lactoferrin für eine Dauer von vier Wochen in der akuten Phase der zystischen Fibrose umfasst.

10. Zusammensetzung zur Verwendung in der Behandlung von zystischer Fibrose und assoziierten Lungeninfektionen gemäss einem der Ansprüche 1 bis 6, **dadurch gekennzeichnet, dass** die besagte Behandlung gemäss einem Dosierungsschema durchgeführt wird, welches die zweimal tägliche Abgabe von 5 ml einer Formulierung mit 500µM an OSCN⁻-Ion und 20mg an Lactoferrin für eine Dauer von vier Wochen in der akuten Phase der zystischen Fibrose umfasst.

11. Zusammensetzung zur Verwendung in der Behandlung von zystischer Fibrose und assoziierten Lungeninfektionen gemäss einem der Ansprüche 1 bis 6, **dadurch gekennzeichnet, dass** die besagte Behandlung gemäss einem Dosierungsschema durchgeführt wird, welches die zweimal tägliche Abgabe von 5 ml einer Formulierung mit 25µM an OSCN⁻-Ion und 1mg an Lactoferrin als Langzeit-Hintergrundtherapie umfasst.

## Claims

1. Composition comprising a synergistic combination of at least one ion selected from the group of hypothiocyanites and/or hypohalites and lactoferrin for use in the treatment of cystic fibrosis and associated pulmonary infections.

2. The composition for use in the treatment of cystic fibrosis and associated pulmonary infections according to claim 1, **characterised in that** the associated pulmonary infections are caused by at least one bacteria chosen in the group constituted by *Burkholderia cepacia, Pseudomonas aeruginosa* and *Staphylococcus aureus.*

3. The composition for use in the treatment of cystic fibrosis and associated pulmonary infections according to any of the preceding claims, **characterised in that** the ion is the hypothiocyanite ion (OSCN⁻).

4. The composition for use in the treatment of cystic fibrosis and associated pulmonary infections according to any of the preceding claims, **characterised in that** the ion is the hypoiodite ion (OI⁻).

5. The composition for use in the treatment of cystic fibrosis and associated pulmonary infections according to any of the preceding claims, **characterized in that** said treatment comprises administration for a local treatment of the pulmonary epithelium of a therapeutically active quantity of a synergistic combination of at least one ion selected from the group of hypothiocyanites and/or hypohalites and lactoferrin.

6. Pharmaceutical formulation designed for the treatment of acute phases of cystic fibrosis, **characterized in that** it comprises 500µM of OSCN⁻ ion and 20mg of lactoferrin.

7. Pharmaceutical formulation designed for the treatment of acute phases of cystic fibrosis, **characterized in that** it comprises 250µM of OSCN⁻ ion and 10mg of lactoferrin.

8. Pharmaceutical formulation designed for the treatment of acute phases of cystic fibrosis, **characterized in that** it comprises 25µM of OSCN⁻ ion and 1mg of lactoferrin.

9. The composition for use in the treatment of cystic fibrosis and associated pulmonary infections according to any of claims 1 to 6, **characterized in that** said treatment is performed according to a dosage regimen that comprises the administration twice daily of 5ml of a formulation comprising 250µM of OSCN⁻ ion and 10mg of lactoferrin for a duration of four weeks in the acute phases of cystic fibrosis.

10. The composition for use in the treatment of cystic fibrosis and associated pulmonary infections according to any of claims 1 to 6, **characterized in that** said treatment is performed according to a dosage regimen that comprises the administration twice daily of 5ml of a formulation comprising 500µM of OSCN⁻ ion and 20mg of lactoferrin for a duration of four weeks in the acute phases of cystic fibrosis.

11. The composition for use in the treatment of cystic fibrosis and associated pulmonary infections according to any of claims 1 to 6, **characterized in that** said treatment is performed according to a dosage regimen that comprises the administration twice daily of 5ml of a formulation comprising 25µM of OSCN⁻ ion and 1mg of lactoferrin as long-term background therapy.
